(19) Europäisches Patentamt
European Patent Office
Office européen des brevets

(11) **EP 4 810 002 A1**

(12) **EUROPEAN PATENT APPLICATION**

(43) Date of publication:
**23.09.2026 Bulletin 2026/39**

(21) Application number: **25165326.7**

(22) Date of filing: **21.03.2025**

(51) International Patent Classification (IPC):
***A61K 8/02*** *(2006.01)* ***A61K 8/34*** *(2006.01)*
***A61K 8/60*** *(2006.01)* ***A61K 8/73*** *(2006.01)*
***A61K 8/9789*** *(2017.01)* ***A61Q 1/06*** *(2006.01)*
***C09D 191/00*** *(2006.01)* ***C09G 3/00*** *(2006.01)*
***C14C 9/02*** *(2006.01)*

(52) Cooperative Patent Classification (CPC):
(C-Sets available)
**A61K 8/0229; A61K 8/022; A61K 8/0254; A61K 8/345; A61K 8/60; A61K 8/731; A61K 8/9789; A61Q 1/06; C08K 5/053; C08L 1/02; C09G 3/00;** A61K 2800/31; A61K 2800/41; A61K 2800/592 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Weidmann Holding AG**
**8640 Rapperswil (CH)**

(72) Inventors:
- **Mettler, Thomas**
  **8640 Rapperswil (CH)**
- **Hilty-Vancura, Florentine**
  **8640 Rapperswil (CH)**
- **Westrick, Manon**
  **8640 Rapperswil (CH)**
- **Zanella, Serena**
  **8640 Rapperswil (CH)**
- **Ferrari, Ludovica**
  **8640 Rapperswil (CH)**

(74) Representative: **Kailuweit & Uhlemann Patentanwälte Partnerschaft mbB**
**Bamberger Straße 49**
**01187 Dresden (DE)**

Remarks:
Amended claims in accordance with Rule 137(2) EPC.

(54) **HEAT RESISTANT SOLID ANHYDROUS COMPOSITION**

(57) The present invention relates to the field of solid anhydrous compositions that are dedicated to be applied on a surface, among others on leather, on metal, on ski soles, and on body surfaces like skin or hair. In particular it relates to the use of a dry mixture as an anti-sweating agent in solid anhydrous compositions, wherein the dry mixture comprises at least one polyol and a fibrous material being a chemically unmodified plant pulp comprising cellulose of natural origin and obtained from plants as an anti-sweating-agent in a solid anhydrous composition, wherein the fibrous material is mechanically obtained from plant pulp, and the plant pulp cellulose and the fibrous material cellulose have not been chemically modified, and the fibrous material comprises more than 10 wt% xylose referred to the total weight of the fibrous material. Moreover, it relates to a solid anhydrous personal care composition comprising the dry mixture as an anti-sweating agent.

10 µm

Fig. 1



Processed by Luminess, 75001 PARIS (FR)

(Cont. next page)

(52) Cooperative Patent Classification (CPC): (Cont.)

C-Sets
**C08L 1/02, C08K 5/053**

## Description

The present invention relates to the field of solid anhydrous compositions that are dedicated to be applied on a surface, among others on leather, on metal, on ski soles, and on body surfaces like skin or hair. In particular it relates to the use of a dry mixture as an anti-sweating agent in solid anhydrous compositions, wherein the dry mixture comprises at least one polyol and a fibrous material being a chemically unmodified plant pulp comprising cellulose , wherein the fibrous material is mechanically obtained from plant pulp, and the fibrous material comprises more than 10 wt% xylose referred to the weight of the fibrous material. Moreover, it relates to a solid anhydrous composition comprising the dry mixture as an anti-sweating agent.

Solid anhydrous or water-free compositions comprise essentially lipids and are used for material care and personal care. In the context of the present invention, material care compositions are compositions used for the care of surfaces from various materials such as leather, shoes, fabrics, metal and ski soles. Personal care relates to the care of skin and/or hair.

It is known from the prior art that material care compositions are available in the form of solid anhydrous compositions. Solid anhydrous compositions for the care of material like leather greases, ski waxes, anti-seizing compositions, e.g. for bicycles, have many advantages when used in solid form. Due to the solid form of the compositions, a thicker and more resistant deposit can be applied on the surfaces. The result is durable protection and greater flexibility of the surface to which it is applied. Leather stays shiny, skis stay smooth, bicycle chains stay flexible. Further advantageously, solid anhydrous or water-free compositions can be transported in a foil or in coated board packaging and thus are very convenient for traveling or practicing sport. In particular solid anhydrous compositions such as shoe grease or solid ski wax with minimal packaging require little space in the bag or pocket.

The main disadvantage of existing solid anhydrous compositions for the care of material of the prior art is that they use to comprise petroleum-based components like paraffin wax and microcrystalline wax and other harmful products like fluorocarbon waxes. Even if petroleum and fluor based waxes have the advantage to have a high melting point (60-70°C), they are very harmful for the environment. For example paraffin wax needs several years to decompose, and releases toxins, some of which can cause cancer and other ailments (Hossain ME, Khan MI, Ketata C, Islam R (2012) "Comparative pathway analysis of paraffin wax and bee wax for industrial application", Journal of Characterization and Development of Novel Materials 1(4):1-13). Fluorocarbon waxes do not degrade at all and end in the watersheds and are carcinogenic.

Therefore, more and more, petroleum-based, fluor-based and other toxic components need to be replaced with plant-based and animal-based lipids whose production and degradation cause less damage to the environment and to human health.

Shoe greases of the prior art comprise mainly animal and plant-based lipids such as beeswax and coconut oil. Nevertheless, plant- and animal-based solid anhydrous compositions are sensitive to heat, wherein when the ambient air is equal to the melting temperature of one of the lipids, the fluid lipid starts separating from the rest of the solid anhydrous composition and migrates on its surface. This phenomenon is called "sweating".

This is an issue for the transport of the plant-based solid anhydrous compositions during the sport activity in summer, or in winter when the material care composition is carried close to the body. Due to global warming and the desire to produce environmentally friendly products, there is the need for solid anhydrous compositions for material care, that do not sweat at warmer temperatures.

In the domain of skin and hair care, most of the personal care compositions comprise water. Solid anhydrous compositions or water-free personal care compositions do not contain water. Consequently, they are microbiologically more stable i.e. they are less contaminable with microorganisms than water-based personal care compositions. Furthermore, anhydrous personal care compositions have a better shelf life than water-based personal care compositions, without requiring preservatives. Preservatives used in water-based personal care compositions can be harmful for the environment, and can also cause damage to the skin, like drying, irritation and allergies. Thus, anhydrous or water-free personal care compositions have the advantage of being ecologically friendly and represent less risk for skin damage. Moreover, due to their solid form, they are also more convenient to use.

Solid anhydrous personal care compositions can be in the form of a stick, a powder, a pressed powder, a tablet, a bar. They can be arranged in a box or in a stick holder from plastic or from coated cardboard. Unlike liquid, gel like or powder personal care composition, solid anhydrous personal care compositions can be applied quickly and precisely to a defined surface of the skin, on hair, on eyebrows or on the lips, without any risk of smudging or spilling. They are particularly convenient for travelling or in the handbag. After application, if necessary, they can be easily blended with a brush, with a sponge or with fingers.

Solid anhydrous personal care compositions such as lipsticks, eyeshadow, blushes, foundation or color cosmetics in solid form comprise mainly a mix of different lipids. Consequently, they are sensitive to heat ("sweating"). The sweating of a solid anhydrous personal care composition results in an inhomogeneous personal care composition with drops on its surface which is inconvenient to handle, and which has lost a part of its original properties.

Consequently, in the domain of material care compositions as well as in the domain of personal care composi-

tions, there is a need for biodegradable solid anhydrous compositions that do not sweat at temperatures between 20°C and 50°C.

Since solid anhydrous compositions comprise mainly lipids, they mix easily with hydrophobic and/or lipophilic compositions. It is known from the state of the art to use hydrophobic rheological modifiers such as mineral or mineral-derived compositions as anti-sweating agent in solid anhydrous composition. Well known mineral and mineral-derived compositions used as anti-sweating agent are among others fumed silica (e.g. Aerosil® 200), or hectorite clay derivative (e.g. Bentone® 38V). The disadvantage of minerals is that they are not renewable. In addition, Bentone® 3V and Aerosil® 200 are chemically modified, which means that their manufacturing requires chemicals. Moreover, the handling of such very fine powder can damage the lungs.

The use of hydrophobic plant-derived compositions as anti-sweating agent is also known. For example, GB 1134170 A discloses that ethyl cellulose, nitrocellulose, cellulose acetate butyrate, and cellulose acetate propionate can be incorporated in nacreous lipstick formulations in order to inhibit sweating of the formulations. But GB 1134170 A discloses only the use of chemically modified cellulose, and as showed in the examples of the present application, the anti-sweating effect of chemically modified cellulose, in particular of ethyl cellulose is limited. In addition, ethyl cellulose, nitrocellulose, cellulose acetate butyrate, and cellulose acetate propionate are cellulose derivatives and require toxic, flammable and/or corrosive solutions such as methyl chloride, propionic and sulfuric acid.

WO 9511000 A1 discloses that ethyl cellulose has also sweat-resistance properties and in particular to retain the oils in the lipstick matrix up to 32°C, when ethyl cellulose is present in an amount of 0.1% to about 20% of the lipstick composition. Ethyl cellulose is a chemically modified plant derived composition obtained by ethylation, which is a chemical process requiring an alkali or an acid. It is hydrophobic; thus it mixes very well with other hydrophobic compositions such as triglycerides and fatty acids. Consequently, ethyl cellulose contributes to stabilizing compositions comprising lipids. Nevertheless, ethyl cellulose has only a limited inhibiting effect on the sweating of anhydrous compositions in a temperature range between 20° and 32°C.

Another disadvantage of ethyl cellulose is that it is not ready biodegradable (Bading M, Olsson O, Kümmerer K (2024) "Analysis of environmental biodegradability of cellulose-based pharmaceutical excipients in aqueous media", Chemosphere 352:141298). This means that less than 60% of the ethyl cellulose meets the required 10-day window defined by "OECD 301 Ready Biodegradability", in the OECD Guidelines for the Testing of Chemicals (1992).

Due to the growing consciousness of the impact of the environment pollution and the impact of rising air temperatures on the flora, the fauna and the human health, it is necessary to produce solid anhydrous compositions which have an enhanced heat resistance, with components that are biodegradable and that are well tolerated by the human skin.

Therefore, it is an object of the present invention to provide a solid anhydrous composition which requires as little chemical processes and modifications as possible and that does not sweat at temperatures between 20 and 40°C.

Surprisingly it has been found, that a dry mixture comprising at least one polyol and a fibrous material, wherein the fibrous material is a chemically unmodified plant pulp comprising cellulose, wherein the fibrous material contains more than 10% xylose referred to the weight of the fibrous material, can be homogeneously mixed to a solid anhydrous composition, in particular a hydrophobic composition, and that the resulting composition has enhanced anti-sweating properties over the prior art at room temperatures up to 45°C. Advantageously, the fibrous material is of natural origin, in particular from plants, and is not chemically modified. Further advantageously, it has been found that a lip stick comprising the dry mixture comprising at least one polyol and a fibrous material as an anti-sweating-agent is harder and has a more intense color.

Without being bound by theory, it is believed that the positive effect observed in this experiment is due to the oil absorption properties of the dry mixture comprising the at least one polyol and a fibrous material, which is a chemically unmodified plant pulp comprising cellulose and more than 10 wt% xylose.

A first aspect of the invention is the use of a dry mixture comprising at least one polyol and a fibrous material as an anti-sweating-agent in a solid anhydrous composition. The fibrous material is a chemically unmodified plant pulp comprising cellulose. In embodiments, the fibrous material comprises micro-scaled fibril agglomerates.

In embodiments, the fibrous material comprises cellulose with a content in the range of 65 wt% to 90 wt% referred to the weight of the fibrous material. The fibrous material of the dry mixture contains more than 10 wt% xylose, in particular more than 15 wt% xylose, referred to the weight of the fibrous material. In embodiments, the fibrous material of the dry mixture contains 10 wt% to 35 wt% xylose, in particular 15 wt% to 25 wt% xylose, referred to the weight of the fibrous material.

In embodiments, the dry mixture comprises fibrous material with a content in the range of 40 wt% to 67 wt%, preferably in the range of 40 wt% to 50 wt%, to the weight of the dry mixture.

In embodiments, the fibrous material is obtained from the pulp of hardwood, wherein the fibrous material contains low levels of lignin. In embodiments, the fibrous material comprises lignin with a content less than 2 wt%, preferably less than 1 wt% referred to the weight of the fibrous material, in particular lignin with a content in the range of 0 wt% to 2 wt%, preferably lignin with a content in the range of 0 wt% to 1 wt% referred to the weight of the fibrous material.

In embodiments, the dry mixture comprises fibrous material comprising micro-scaled fibril agglomerates with an

average length in the range of 500 nm to 1000 μm, preferably in the range of 500 nm to 600 μm and even more preferably in the range of 500 nm to 300 μm. In embodiments, the micro-scaled fibril agglomerates are networks of cellulose fiber structures interconnected with each other. In embodiments, the micro-scaled fibril agglomerates comprise a surface in the range of 40 $m^2/g$ to 450 $m^2/g$, preferably a surface in the range of 50 $m^2/g$ to 400 $m^2/g$, more preferably a surface in the range of 60 $m^2/g$ to 400 $m^2/g$, more preferably a surface in the range of 80 $m^2/g$ to 350 $m^2/g$.

In embodiments, the dry mixture comprises the polyol and fibrous material with a weight ratio in the range of 0.5 to 1.5, preferably in the range of 0.7 to 1.0, for example in the range of 0.8, 0.85, 0.90, 0.95 or 0.99.

In embodiments, the dry mixture has a water activity that is lower than 0.6 at room temperature. In embodiments, the dry mixture has a water activity in the range of 0 to 0.6 at room temperature.

In embodiments, the fibrous material is a plant pulp obtained from Eucalyptus tree, beech tree, birch tree, populus tree, in particular aspen or poplar; and/or oak tree, preferably from Eucalyptus Urograndis tree.

In embodiments, the dry mixture comprises at least one polyol which is selected from the group consisting of glycerol, erythritol, threitol, pentaerythritol, xylitol, ribitol, arabinitol, mannitol, sorbitol, allitol, altritol, galactitol, glucitol, iditol, and xylitol. In embodiments, the dry mixture comprises a mixture of polyols selected from the group consisting of glycerol, erythritol, threitol, pentaerythritol, xylitol, ribitol, arabinitol, mannitol, sorbitol, allitol, altritol, galactitol, glucitol, iditol, and xylitol.

In embodiments, the dry mixture is in powder form, granulated form or in flake form or the like. In embodiments, the dry mixture is in powder form, or in flake form.

In embodiments, the solid anhydrous composition is a personal care composition, preferably a personal care composition selected from a skin care, a color cosmetic, and a hair care, in particular a lipstick, an eyeshadow, a brow cream, a foundation, a sunscreen composition, a solid perfume or a deodorant. In embodiments, the solid anhydrous composition is a material care composition, preferably a material care composition selected from leather greases, ski waxes, wood furniture protection and anti-seizing compositions, in particular for bicycles. In embodiments, the material care composition is used to be applied to leather, metal, plastics, wood or rubber.

Another aspect of the invention is a solid anhydrous composition, wherein the solid anhydrous composition comprises:

- 20 wt% to 50 wt% lipids that are solid at room temperature,
- 30 wt% to 80 wt% lipids that are fluid at room temperature,
- at least 0.1 wt%, preferably in the range of 0.1 wt% to 5 wt%, more preferably in the range of 1 wt% to 3 wt%, of a dry mixture comprising at least one polyol and a fibrous material, wherein the fibrous material is a chemically unmodified plant pulp comprising cellulose,

wherein the fibrous material contains more than 10 wt% xylose referred to the weight of the fibrous material, preferably 10 wt% to 35 wt% xylose referred to the weight of the fibrous material. In embodiments, the fibrous material comprises micro-scaled fibril agglomerates.

In embodiments, the solid anhydrous composition comprises lipids that are solid at room temperature and lipids that are fluid at room temperature in a ratio in the range of 1:4 to 5:3.

Suitably, the components of the solid anhydrous composition, in particular the dry mixture, the lipids that are solid at room temperature and the lipids that are fluid at room temperature, add up to 100%.

In embodiments, the solid anhydrous composition comprises at least one colorant in the range of 5 wt% to 15 wt% referred to the weight of the solid anhydrous composition.

In embodiments, the solid anhydrous composition comprises at least one functional ingredient. In embodiments, the solid anhydrous composition comprises at least one functional ingredient in the range of 0.15 wt% to 3 wt% referred to the weight of the solid anhydrous composition.

Furthermore, a method for preparing a solid anhydrous composition, in particular a solid anhydrous composition according to the invention, is provided, wherein the method comprises at least the following steps:

a) Heating up lipids that are solid at room temperature to a temperature in the range of 75°C to 90°C,
b) Mixing lipids that are fluid at room temperature to the heated lipids of step a) and cooling down to a temperature in the range of 70°C to 80°C,
c) Adding a dry mixture comprising at least one polyol and a fibrous material to the lipids, wherein the fibrous material is a chemically unmodified plant pulp comprising cellulose, wherein the fibrous material contains more than 10 wt% xylose referred to the weight of the fibrous material,
d) Mixing with a homogenizer until a homogeneous composition is obtained, and
e) Cooling down the homogenous composition.

In embodiments, the method is carried out with the steps in the order a), b), c), d), and e).

In embodiments, in step a) the lipids that are solid at room temperature are heated up under stirring. In embodiments, in step b) the cooling down is carried out under stirring.

In embodiments, after step a), b) and/or c) at least one colorant and/or at least one functional ingredient are added to the lipids.

In embodiments, at least one colorant and/or at least one functional ingredient are added to the lipids after step b). In embodiments, the dry mixture is added afterwards to the mixture of lipids and colorant and/or functional ingredient.

In embodiments, the colorant and/or functional ingredient are added to the dry mixture in step c) and added to the lipids.

In embodiments, the dry mixture is added to the mixture of lipids after step b). In embodiments, at least one colorant and/or at least one functional ingredient are added to the mixture after step c).

In embodiments, after step c) mixing with a homogenizer is carried out.

In embodiments, after step d) shaping is carried out. In embodiments, the shaping of the mixture is carried out by filling the mixture into a container, in particular a tube or a box, or by pouring the mixture into a mold.

In embodiments, after step d) the mixture is cooled down by incubation at a temperature in the range of 0°C to 20°C, preferably a temperature in the range of 0°C to 10°C, more preferably a temperature of about 4°C. In embodiments, after step d) the mixture is cooled down for at least 10 minutes, preferably for 10 minutes to 60 minutes.

In embodiments, after step e) the mixture is taken out of the mold. In embodiments, after step e) the mixture is taken out of the mold and incubated at room temperature for about 24 hours.

All components used herein are expressed as a percentage by weight of the composition.

- FIG. 1 shows a view of a part of a micro-scaled fibril agglomerate obtained by means of an electron microscope at a magnification of 10’000.
- FIG. 2 shows pictures of four lipsticks, Nr. 1, Nr. 2, Nr. 3 and Nr. 4 at 25°C (room temperature), before heat exposure.
- FIG. 3 shows pictures of four lipsticks Nr. 1, Nr. 2, Nr. 3 and Nr. 4 after having been exposed to a temperature of 45°C for 60 minutes.
- FIG. 4 shows pictures of three lipsticks, Nr. 5, Nr. 6 and Nr. 7 at 25° C (room temperature), before heat exposure.
- FIG. 5 shows pictures of three lipsticks, Nr. 5, Nr. 6 and Nr. 7 after having been exposed to a temperature of 45°C for 15 minutes.
- FIG. 6 shows pictures of three lipsticks Nr. 5, Nr. 6 and Nr. 7 after having been exposed to a temperature of 45°C for 30 minutes.
- FIG. 7 shows pictures of three lipsticks Nr. 5, Nr. 6 and Nr. 7 after having been exposed to a temperature of 45°C for 60 minutes.
- FIG. 8 shows pictures of three lipsticks Nr. 5, Nr. 6 and Nr. 7 after having been exposed to a temperature of 45°C for 120 minutes.

ANTI-SWEATING AGENT

In the context of the present invention, an anti-sweating agent is a component of a solid anhydrous composition, preventing the excretion of liquid onto the surface of a solid anhydrous composition comprising lipids, when exposition to heat up to 50°C.

SOLID ANHYDROUS COMPOSITION

In the context of the present invention, a solid anhydrous composition is a composition that is solid at room temperature and does not comprise water or wherein the water content is low, in particular comprising a water activity of lower than 0.6. The solid anhydrous composition comprises lipids and is dedicated to being applied on a surface, by hand or by means of a container.

In embodiments, the solid anhydrous composition is in the form of a stick, a powder, a pressed powder, a tablet or a bar.

In embodiments, the solid anhydrous composition is arranged in a re-usable container, such as a box or a stick holder made of plastic or coated cardboard.

Solid anhydrous compositions according to the present invention can be applied quickly and precisely on a defined surface of an object, on the skin, on hair, on eyebrows or on the lips, without any risk of smudging or spilling. In embodiments, the solid anhydrous composition is a leather care composition, a ski gliding composition, an anti-seizing composition, or a personal care composition.

In the context of the present invention the personal care composition is preferably selected from a skin care, a hair care, a color cosmetic, a solid perfume or a deodorant, in particular a lipstick, a lip balm, an eyeshadow, a brow cream, a foundation or a sunscreen composition.

They are particularly convenient for travelling or in the handbag. After application, if necessary, they can be easily blended with a brush, with a sponge or with fingers.

In embodiments, the solid anhydrous composition comprises 20 wt% to 50 wt% lipids that are solid at room temperature, 30 wt% to 80 wt% lipids that are fluid at room temperature, and at least 0.1 wt% of a dry mixture comprising at least one polyol and a fibrous material, wherein the fibrous material is a chemically unmodified plant pulp comprising cellulose, wherein the fibrous material contains more than 10 wt% xylose referred to the weight of the fibrous material, preferably 10 wt% to 35 wt% xylose referred to the weight of the fibrous material. In embodiments, the fibrous material comprises micro-scaled fibril agglomerates. The fibrous material, in particular the plant pulp cellulose, has not been chemically modified.

DRY MIXTURE

In the context of the present invention, "dry" mixture or "dry" powder has traces of moisture, in particular a water activity lower than 0.6, preferably in the range of 0 to 0.6, which does not allow any bacteria growth. As used herein, the term "water activity" refers to the ratio of its vapor pressure to the vapor pressure of water at the same temperature and is a measure of the "available" or "active" water. Consequently, the dry mixture has the advantage of being microbiologically stable which implies a long shelf life of the dry mixture and thus makes the use of preservative unnecessary. The absence of toxic preservatives in the dry mixture makes it easy for people to handle without risk to their lungs, skin and eyes.

The dry mixture comprising at least one polyol and fibrous material, as used in the context of the present invention can be obtained by performing the process disclosed in EP 3606500 B1.

In embodiments, the fibrous material is obtained by performing at least the steps of:

a) comminuting dry pulp by mechanical means;
b) dispersing said dry comminuted pulp in a liquid, preferably a protic liquid, more preferably a protic liquid comprising water, even more preferably water; and
c) further comminuting the pulp dispersed in the liquid to form a mixture of a fibrous material comprising micro-scaled fibril agglomerates and the liquid, in particular by means of a mineral material. The result of the further comminution according to step c) is a gel-like mixture comprising the liquid and a fibrous material comprising micro-scaled fibril agglomerates. This mixture can be used as a basis to prepare the dry mixture used as an anti-sweating agent according to the present invention.

In embodiments, the dry mixture is obtained by further processing the mixture comprising fibrous material comprising the micro-scaled fibril agglomerates and a liquid obtained after step c) with at least the following steps: Adding at least one polyol to the mixture of fibrous material comprising micro-scaled agglomerates and the liquid provided in step c) to form a mixture and Drying the mixture by applying a drying method involving heat, electromagnetic waves and/or vacuum or the like, to remove the liquid and thus form a dry mixture.

In embodiments, the dry mixture has a solid content in the range of 70 wt% to 100 wt%, more preferably in the range of 80 wt% to 99 wt%, even more preferably in the range of 90 wt% to 97 wt%, referred to the total weight of the dry mixture. In the context of the present invention, "solid content" refers to the proportion of fibrous material and polyol in the dry mixture. The solid content of the dry mixture can be determined by means of standard EN 20638 of September 1993. In embodiments, the dry mixture is in the form of a powder or flakes.

FIBROUS MATERIAL

The dry mixture used in the present invention comprises fibrous material. The fibrous material that is plant pulp, which is mechanically processed.

As used herein, the term "plant pulp" refers to a cellulose-containing material obtained by chemical or mechanical means from a material selected from wood, annual plant, agriculture crops, fruits, e.g. citrus fruits or apples; cereals, e.g. wheat or maize; grass or fiber containing vegetables, such as peas, pulses or carottes; or cellulose-containing textiles by pulp production processes well known from the person skilled in the art, such as sulphate, sulphite or soda processes.

In the context of the present invention, the fibrous material is obtained from plant pulp that has not been treated with any chemicals resulting in chemical modification previously. Thus, the plant pulp and the cellulose comprised within the plant pulp are not chemically modified. For example, no carboxymethylation, no ethylation, and no TEMPO (2,2,6,6-Tetramethylpiperidin-1-yl)oxyl)-oxidation has been carried out. As used herein, the term "cellulose" refers to a polysaccharide consisting of a linear chain of several hundred to many thousands of $\beta(1\rightarrow4)$ linked D-glucose units.

If wood is used as the original cellulose-containing material, it is usually first reduced to wood chips and then, the cellulose is extracted e.g. by purely mechanical means or by means of a purely chemical treatment or by means of a combination thereof, to the pulp. Thus, the wood is delignified and frayed, meaning that the cellulose fibers are extracted

from the original wood structure. In the plant pulp process, xylose remains with the cellulose.

The content of lignin in the pulp and of the fibrous material may be assessed by methods known to the art, such as, for example, the method according to the standard TAPPI T222 om-02 of June 16, 2006.

In the method for producing the fibrous material (see paragraph "DRY MIXTURE"), both comminuting steps a) and c) and the dispersion step b) are mechanical processes. The liquid in which the dried comminuted pulp is dispersed in step b) does not chemically modify the pulp. Preferably the liquid is water. Thus, the fibrous material of natural origin is obtained mechanically from plant pulp, wherein neither the plant pulp cellulose nor the fibrous material have been chemically modified. In step a) the pulp fibers are shortened and in the step c) the shortened dispersed fibers are separated from each other's.

In embodiments, the fibrous material comprises micro-scaled fibril agglomerates. In embodiments, the fibrous material comprises at least 10 wt%, preferably at least 20 wt%, more preferably at least 40 wt%, even more preferably at least 60 wt%, even more preferably at least 80 wt%, even more preferably at least 90 wt%, even more preferably at least 95 wt%, most preferably 100 wt%, micro-scaled fibril agglomerates.

The average length of the micro-scaled fibril agglomerates comprised by the fibrous material is in the range of 500 nm to 1000 μm, more preferably in the range of 500 nm to 600 μm, and even more preferably in the range of 500 nm to 300 μm. In embodiments, the average length is determined according to standard ISO 13322-2, 1. Edition of November 1, 2006.

In embodiments, the $D_{90}$ value of the length of the micro-scaled fibril agglomerates comprised in the fibrous material is in the range of 1 μm to 200 μm, preferably in the range of 30 μm to 150 μm, more preferably in the range of 40 μm to 140 μm. The $D_{90}$ value of the length of the micro-scaled fibril agglomerates indicates the maximal length of 90% of the fibril agglomerates. In embodiments, the $D_{90}$ value of the thickness of the micro-scaled fibril agglomerates comprised in the fibrous material is in the range of 0.5 μm to 20 μm, preferably in the range of 1 μm to 10 μm, more preferably in the range of 1 μm to 8 μm. The $D_{90}$ value of the thickness of the micro-scaled fibril agglomerates indicates the maximal thickness of 90% of the fibril agglomerates. The calculation of the $D_{90}$ value is known by the person skilled in the art. In embodiments, the length and/or thickness is determined by means of the standard ISO 13322-2, 1. Edition of November 1, 2006. In embodiments, the water retention value of the fibrous material is in the range of 100 wt% to 700 wt%, preferably in the range of 150 wt% to 600 wt%, more preferably in the range of 170 wt% to 500 wt%. In embodiments, the water retention value is determined based on mechanical press dewatering over a sieve. In embodiments, the water retention value is determined based on mechanical press dewatering over a sieve using a mechanical press, in particular a FiberXPress (VOITH). In embodiments, the water retention value is determined according to Wolfinger (Wolfinger T (2016) "Dreidimensionale Strukturanalyse und Modellierung des Kraft-Dehnungsverhaltens von Fasergefügen", TU Dresden, Fakultät Umweltwissenschaften, Dissertation).

In embodiments, the fibrous material comprising the micro-scaled fibril agglomerates comprises a surface in the range of 40 $m^2/g$ to 450 $m^2/g$, preferably a surface in the range of 50 $m^2/g$ to 400 $m^2/g$, more preferably a surface in the range of 60 $m^2/g$ to 400 $m^2/g$, more preferably a surface in the range of 80 $m^2/g$ to 350 $m^2/g$. Personal care compositions comprising such a fibrous material have especially good properties. For the measuring of said surface, nitrogen adsorption measuring is carried out. In embodiments, the sample is dried with ethanol and/or a temperature of at least 105°C before the nitrogen adsorption measurement. In embodiments, the following method comprising the steps "Sample preparation I", "Sample preparation II" and "Performance of the nitrogen adsorption measuring / calculation of the surface of the fibrous material comprising the micro-scaled fibril agglomerates" is used:

Sample preparation I

About 20 g of a fiber dispersion comprising 2 wt% of the fibrous material comprising the micro-scaled agglomerates are weighted into a 50 ml Falcon tube. Ethanol (94%) is added till a volume of 50 ml is reached. After that, the content of the Falcon tube is mixed by means of a vortex mixer. After that, the sample is centrifugated by means of a centrifuge (e.g. Typ Hettich Rotina 380 with 6-way angle rotor 45°) at 5000 $min^{-1}$ for 10 minutes. After that, the supernatant is removed and again ethanol (94%) is added in the falcon tube. After that, the content of the Falcon tube is resuspended and again centrifugated with the same parameters as mentioned above. This procedure is repeated 5 times. Subsequently, the residual moisture of the sample is determined by drying the sample by means of supercritical $CO_2$ in a device such as a Autosamdri®-931 (Tousimis).

Sample preparation II

The sample, which is dewatered according to the method according to sample preparation I, is weighted into a glass tube. Before weighing the sample into the glass tube, the glass tube is dried and weighed when it is empty. After that, the sample comprised by the glass tube is degassed for at least 24 hours at a temperature of at least 105°C.

Performance of the nitrogen adsorption measuring / calculation of the surface of the fibrous material comprising the micro-scaled fibril agglomerates:

The glass tube comprising the degassed sample, wherein the degassing was performed by means of sample preparation II, is now arranged in a suitable measuring device, such as a Micromeritics 3Flex Version 3.01, in order to determine the nitrogen sorption isotherm. The sample mass is for example 0.0777 g, but the sample mass can deviate from said sample mass because of the type of measuring device or because of the specific sample. The nitrogen and the helium which are used for the performance of the measuring have a purity of 99,999 %. The result of the measuring is calculated to one decimal place in $m^2/g$ ($m^2/1g$). The calculated value of the result is based on the BET-method according to Brunauer, Emmett and Teller which is known by person skilled in the art.

CHEMICALLY NOT MODIFIED

The fibrous material and the plant pulp used for the preparation of the fibrous material are not chemically modified. Its chemical constitution and its molecular structure have not been changed. This means that the cellulose in the fibrous material has the same chemical state as the cellulose in the pulp. With the same chemical state is meant that the hydroxyl groups of the cellulose of the fibrous material have no other covalent bound than the one existing in the pulp.

XYLOSE

As used herein, the term "Xylose" refers to a sugar, in particular a monosaccharide of the aldopentose type, which can be isolated from wood. Furthermore, the xylose content of the fibrous material originates from the plants used in the production of the pulp used for the dry mixture.

That means that no xylose has been added as an additive at any time. The xylose has not or not completely been removed from the plant pulp during the pulping process. The xylose of the fibrous material of the dry mixture, i.e. the xylose being already part of the plant raw material, presents the advantage to be better and more homogeneously dispersed in the fibrous material. Consequently, it requires a smaller amount of xylose in the fibrous material to achieve similar effects than a fibrous material wherein the xylose has been added as an additive.

Xylose in the fibrous material leads to a particularly smooth and silky feel of the fibrous material. Surprisingly, it was found that a higher xylose content in the microscaled agglomerates provides a better stabilization for the fibrous material, than respective micro-scaled fibril agglomerates with no xylose or a low xylose content. A homogenous fibrous material is obtained leading to the smoother and silky feeling.

In embodiments, the amount of xylose comprised by the fibrous material is measured by hydrolysis and subsequent carbohydrate analysis, in particular using a two-stage sulfuric acid hydrolysis of the fibrous material, in particular a sulfuric acid concentration of 72% at 30°C and a sulfuric acid concentration of 2.5% at 120°C; and a characterization of the soluble components using HPLC chromatography (High Pressure Liquid Chromatography). In embodiments, the amount of xylose comprised by the fibrous material is measured according to Wolfinger (Wolfinger T (2016) "Dreidimensionale Strukturanalyse und Modellierung des Kraft-Dehnungsverhaltens von Fasergefügen", TU Dresden, Fakultät Umweltwissenschaften, Dissertation).

MICRO-SCALED FIBRIL AGGLOMERATES

In embodiments, the fibrous material comprises micro-scaled fibril agglomerates, which means that the individual fibers and in particular the microfibrils of the original cellulose material are comminuted and are present at least partially or completely separated from each other, wherein the separated microfibrils in particular form fibril agglomerates due to mutual association. The microfibrils being present within the fibril agglomerates are completely separated from the original fiber structure of the cellulose and are interconnected with each other due to mutual adherence, such that they form a common structure, in particular a network.

Within the micro-scaled fibril agglomerates, the individual micro fibrils are strongly interacting, which means that the dissociation of the fibrils constituting the agglomerates from each other would require the use of e.g. a high-pressure homogenizer, typically by passing a dispersion of a liquid, which preferably is an aqueous medium, and of the fibrous material comprising the micro-scaled fibril agglomerates several times through said high-pressure homogenizer. Such high-pressure homogenizer conditions are usually not applied to the personal care compositions in the context of the present invention.

The fibrous micro-scaled fibril agglomerates are visible in the fine-comminuted fibrous material, and in the dried and re-dispersed fibrous material. This is measurable by means of the standard ISO 13322-2, 1. Edition of November 1, 2006.

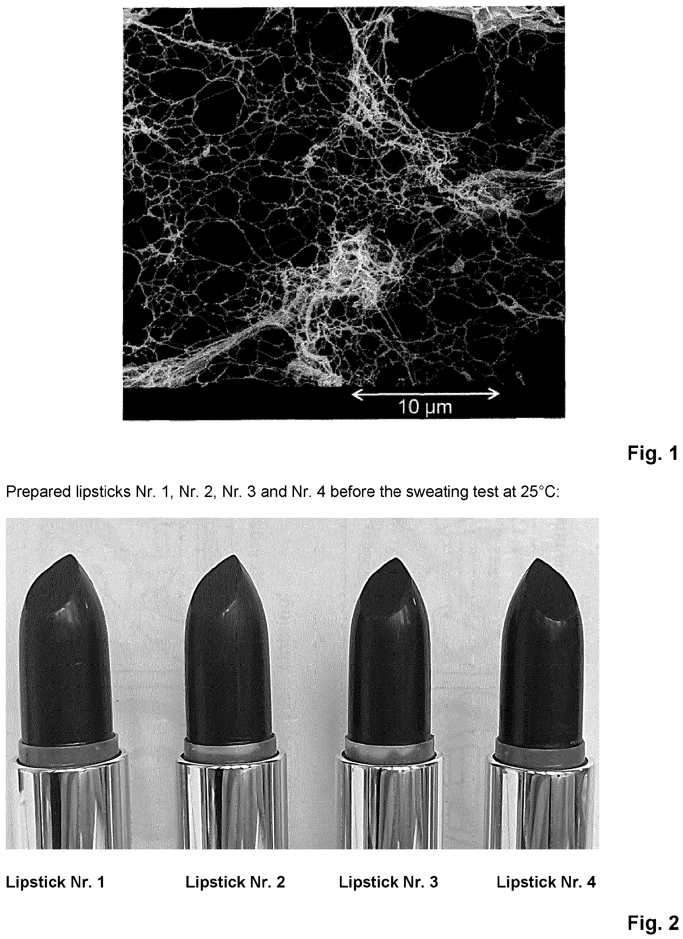

Fig. 1 shows an exemplary view of a part of a micro-scaled fibril agglomerate which is present in the fine-

comminuted fibrous material, and in the dried and re-dispersed fibrous material. Fig. 1 is obtained by an electron microscope at a magnification of 10'000. The typical network-structure being present inside of the micro-scaled fibril agglomerate can clearly be seen. The network is formed by a plurality of comminuted fibrils which are interconnected. The part of the micro-scaled fibril agglomerate is substantially, in the view of Fig. 1, even completely, free of visible isolated fibrils, because the fibrils are only present in reduced sizes and are bound in the networks forming the micro-scaled fibril agglomerates.

POLYOL

In embodiments, the polyol added to the fine comminuted mixture is skin compatible and does not cause any chemical modification of the fibrous material. In the context of the present patent application, "skin compatible" means that it does not cause any itching, burning, stinging, heat, redness, blistering, swelling or the like of the skin. In embodiments, the at least one polyol is selected from the group consisting of glycerol, erythritol, threitol, pentaerythritol, xylitol, ribitol, arabinitol, mannitol, sorbitol, allitol, altritol, galactitol, glucitol, iditol, and xylitol.

The addition of at least one polyol to the mixture of fibrous material allows the preservation of the micro-fibril structures as well as the micro-scaled fibril agglomerates during drying. The polyol shields the hydroxyl groups of the micro-scaled cellulose agglomerates and thus, it prevents the formation of direct strong irreversible connections between the micro-scaled fibril agglomerates. The weight ratio of polyol to fibrous material is in the range of 0.5 to 1.5, preferably in the range of 0.7 to 1.0, for example 0.8, 0.85, 0.90, 0.95 or 0.99. Advantageously, the dry mixture comprising a polyol and a fibrous material with micro-scaled agglomerates, wherein the fibrous material contains at least 10 wt% xylose, preferably 10 wt% to 35 wt% xylose referred to the weight of the fibrous material, can be well re-dispersed in water-based compositions. In particular, once re-dispersed in a water-based composition, the dry mixture recovers a similar BET surface area and a similar viscosity as the mixture of fibrous material with micro-scaled agglomerates, more than 10% xylose, preferably 10 wt% to 35 wt% xylose referred to the weight of the fibrous material, and a liquid before the drying step.

ROOM TEMPERATURE

As used herein, the term "room temperature" refers to a temperature in the range of 20°C to 25°C, preferably about 25°C, more preferably of 25°C.

LIPIDS

In embodiments, the anhydrous composition comprises skin compatible lipids. A skin compatible lipid is defined herein as a lipid that is fluid, semi-solid, or solid at room temperature that is deemed safe for use in cosmetics being either inert to the skin or actually beneficial. Lipids useful herein may include oils and waxes and plant butter. In embodiments, skin compatible lipids include ester lipids, hydrocarbon lipids, silicone lipids, interesterified, non-fractionated triglyceride, plant-based waxes, animal-based waxes and mineral waxes.

Ester lipids have at least one ester group in the molecule. In embodiments, ester lipids are fatty acid monoester or polyester. In embodiments, ester lipids are selected from the group consisting of cetyl octanoate, octyl isonanoanate, myristyl lactate, cetyl lactate, isopropyl myristate, myristyl myristate, isopropyl palmitate, isopropyl adipate, butyl stearate, decyl oleate, glycerol monostearate, alkyl lactate, alkyl citrate, alkyl tartrate, sucrose ester, sucrose polyesters and sorbitol ester.

In embodiments, lipids are interesterified, non- fractionated triglycerides, such as plant based butters selected of but not limited to Shea butter, Shorea Stenoptera seed butter, Cocoa seed butter and Astrocaryum murumuru seed butter.

In embodiments, lipids comprise triglycerides and modified triglycerides. In embodiments, lipids are vegetable oils, preferably selected from the group consisting of jojoba, soybean, canola, sunflower, safflower, rice bran, avocado, almond, olive, sesame, persic, castor, coconut, and mink oils. Synthetic triglycerides can also be employed provided they are fluid at room temperature. Modified triglycerides include ethoxylated and maleated triglyceride derivatives provided they are liquids.

In embodiments, lipids that are fluid at room temperature are vegetable and algae oils, preferably selected from the group consisting of rapeseed oil, sunflower oil, olive oil, argan oil, apricot oil, jojoba oil, aloe vera oil, ricinus oil, grape seed oils and hydrogenated castor oil. Fluid lipids have the advantage of having an emollient function. Emollients smooth and hydrate the skin.

In embodiments, lipids that are fluid at room temperature are esters, preferably selected from the group consisting of isopropyl myristate, methylheptyl isostearate, isononyl isononanoate, dicarpylyl carbonate and coco-caprylate.

In embodiments, skin compatible lipids are fluid and semi-solid hydrocarbons. In embodiments, lipids are linear and branched oils such as liquid paraffin, squalene, squalane, mineral oil or low viscosity synthetic hydrocarbons, such as

polyalphaolefin (PureSyn™ polyalphaolefins (PAO), ExxonMobil) , and polybutene, e.g. Panalane® or Indopol®. Light (low viscosity), highly branched hydrocarbon oils are also suitable.

In embodiments, the liquids are selected from the group comprising mineral oils, petrolatum, hydrogenated isoparaffins, hydrogenated polyisobutylene, and silicone oils. In embodiments, the lipid is petrolatum. Petrolatum is a semi-solid hydrocarbon material. Advantageously, its state of aggregation can be controlled both in production and by the formulator through blending with other oils.

In embodiments, the lipids are silicone based. In embodiments, the lipids are selected from the group consisting of linear and cyclic polydimethyl siloxanes, organofunctional silicones (alkyl, alkyl aryl, copolyol amino), and amino silicones.

In embodiments, the lipids are liquid fatty acids. In embodiments, the liquid fatty acids have from about 10 to about 30 carbon atoms. These fatty acids can be straight or branched chain acids and can be saturated or unsaturated. In embodiments, the lipids are selected from the group consisting of oleic acid, linoleic acid, isostearic acid, linolenic acid, ethyl linolenic acid, arachidonic acid, ricinolic acid, and mixtures thereof.

In embodiments, the lipids are esters of long-chain fatty alcohols with long-chain fatty acids, preferably waxes, plant-derived waxes, animal-derived waxes or mineral-derived waxes.

In embodiments, waxes are plant-derived waxes, preferably selected from the group consisting of rice bran wax, Candelilla wax, sunflower wax, Carnauba wax, Euphorbia Cerifera Wax, tribehenin wax, bayberry wax, jojoba wax, orange wax, castor wax, soy wax, rhuswax, sugarcane wax, Rosa damascena flower wax; or animal-based waxes preferably selected from the group consisting of beeswax, spermaceti wax, Chinese wax, wool wax and shellac wax.

In embodiments, the lipids are petroleum-derived waxes, preferably paraffin wax, petrolatum or microcrystalline wax; or mineral waxes, preferably montan wax, ceresin wax or ozokerite; and synthetic derivatives of natural waxes.

SOLID LIPIDS

In the context of the present invention, solid lipids are lipids as described in the present application under "LIPIDS" and are characterized in that they have a melting temperature above 38°C and thus are in solid or semi-solid form at room temperature. Further examples of solid lipids can comprise but not exclusively waxes and/or plant base butters. Waxes are preferably plant-derived waxes (e.g. rice bran wax, Candelilla wax, sunflower wax, Carnauba wax, Euphorbia Cerifera Wax, tribehenin wax, bayberry wax, jojoba wax, orange wax, castor wax, soy wax, rhuswax, sugarcane wax, Rosa damascena flower wax), or animal-based waxes (e.g. beeswax, spermaceti wax, Chinese wax, wool wax, shellac wax), but can be also petroleum-derived waxes (e.g., paraffin wax, petrolatum, microcrystalline wax), or mineral waxes (e.g., montan wax, ceresin wax, ozokerite) and synthetic derivatives of natural waxes.

Plant-based butter can be Shea butter, Shorea Stenoptera seed butter, Cocoa seed butter or Astrocaryum murumuru seed butter.

FLUID LIPIDS

In the context of the present invention, fluid lipids are lipids as described in the present application under "LIPIDS" and are characterized in that they are fluid at room temperature. Further examples of fluid lipids are vegetable and algae oils, such as rapeseed oil, sunflower oil, olive oil, argan oil, apricot oil, jojoba oil, aloe vera oil, ricinus oil, grape seed oils, hydrogenated castor oil, or the like. Fluid lipids have the advantage of having an emollient function. Emollients smooth and hydrate the skin.

Fluid lipids that are also useful for the sake of the present invention are fluid lipids that may be selected from the group comprising mineral oils, hydrogenated isoparaffins, hydrogenated polyisobutylene, and silicone oils.

Other fluid lipids used in the context of the present invention can be esters, such as isopropyl myristate, methylheptyl isostearate, isononyl isononanoate, dicarpylyl carbonate, coco-caprylate.

FUNCTIONAL INGREDIENTS

In embodiments, the solid anhydrous composition comprises one or more functional ingredients, selected from the group consisting of cleansing ingredients, texturing ingredients, softening ingredients, emollients, hydrating ingredients, lubricating ingredients, smoothening ingredients, soothing and relaxing ingredients, exfoliating ingredients, cell renewal and anti-aging ingredients, draining ingredients, remodeling ingredients, free-radical scavengers, structuring ingredients, anti-oxidant, decorative ingredients, skin levelling ingredients, epilating ingredients, whitening ingredients, deodorizing ingredients, antibacterial or bacteriostatic ingredients, biological preservatives.

In embodiments, the one or more functional ingredient is selected from the group comprising synthetic polymers, natural polymers, solvents, mineral and vegetal oils, surfactants, fatty acids and fatty alcohol esters, in particular C10 to C24 fatty acids and their salts; C10 to C24 fatty alcohols, alpha and beta hydroxy acids and their salts, salicyclic acid and its

salts, dicarboxylic acids and their salts, pyrrolidone carboxylic acid, proteins and peptides, collagen, glycolipides, phospholipides, sphingolipides, steroids, in particular sterols; glycerine ethoxylate, calcium glyconate, polidocanol, urea, allantoin, caffein, flavonoids and isoflavonoids, polyphenols, anthocyanins, minerals, colorants such as organic or inorganic dyes and pigments, vitamins and their derivatives, terpenes and their derivatives, sesquiterpenes and their derivatives, triterpenes and their derivatives, ubiquinones, sequestering ingredients, waxes and butters, carbohydrates and sugar alcohols, and their derivatives, mineral and vegetal particulates, in particular plant extracts, juices, essential oils and oil or wax-based fragrances.

In embodiments, the synthetic or natural polymer is a hydrophilic or hydrophobic polymer. In embodiments, the synthetic or natural polymer is a copolymer or a terpolymer selected from the group consisting of vinylpyrrolidone/acrylate copolymers, copolymers and cross-polymers derived from alkyl (meth)acrylates, (meth)acrylic acids and acrylamidodimethyltauric acid and their salts, vinylpyrrolidone/acrylamido alkylsulphonic acid copolymers and their salts; xanthan gum, dehydroxanthan gum, guar gum, gum Arabic, Accacia gum, Sclerotium gum, Ceratonia siliqua gum; pullulans, glucans, glycoaminoglycanes, carraghenans, pectins, alginates, hyaluronic acid and its salts, sodium hyaluronate cross-polymers, chitosan, and mixtures thereof.

In embodiments, solvents are selected from the group consisting of alcohols, such as ethanol, propanol and isopropanol; polyols, such as 2-ethane diol, 1,2-propane diol, 1,3-propane diol, dipropylene glycol, 1,4-butane diol, glycerol, pentaerythritol, 1,4-butane diol, 1,2-butane diol, 1,2-pentane diol, 1,2-hexane diol, 1,2-heptane diol or 1,2-octane diol; glycol ethers and esters, such as dipropylene glycol methyl ether acetate, tripropylene glycol methyl ether, dipropylene glycol methyl ether, dipropylene glycol n-butyl ether or 3-methoxy-3-methyl-1-butanol; isosorbide dimethyl ether and (2,2-dimethyl-1,3-dioxolan-4-yl)methanol.

In embodiments, the surfactant is an anionic surfactant, preferably selected from the group consisting of C10 to C22 alkylester sulphonates, such as alpha-sulpho fatty acid methyl esters and ethyl esters; alkyl sulphates, such as sodium dodecyl sulphate; alkyl ether sulphates obtained by ethoxylation of an alkyl alcohol followed by sulphonation using sulphur trioxide; sodium alkyl isethionate; sodium alkoyl sarcosinate, such as sodium dodecanoyl(methyl)aminoacetate; sodium laurylglucosides hydroxypropylsulphonate; potassium laureth phospate; or a cationic surfactant, preferably selected from the group consisting of quaternized long chain alkyl ammonium halides, such as distearyldimethylammonium chloride, behenyltrimethylammonium chloride, palmitamidopropyldimethyl-ammonium chloride; linoleamidopropyl ethyldimethyl ammonium ethylsulphate, lecithin and lysolecithin; or a cationogenic surfactant, preferably selected from the group consisting of fatty amines, such as lauriminopropyldimethyl amine, tridecyl amine, N-oleyl-1,3-propane diamine, and ethoxylated fatty amines, such as ethoxylated N-tallow-1,3-propanediamine; or a zwitterionic surfactant, derived from the reaction between compounds having alkyl quaternary ammonium, phosphonium, or sulfonium groups and compounds having carboxyl, sulfonate, sulfate, succinate, phosphate or phosphonate groups, such as coco dimethylcarboxymethyl betaine, lauryl dimethyl carboxymethyl betaine, stearyl bis-(2-hydroxyethyl) carboxymethyl betaine; amidoalkyl, sulfoalkyl and alkyl amidosufo betaines, such as cocoamidopropyl betaine, cocodimethylsulfopropyl betaine, lauryl dimethyl sulfoethyl betaine, and lauryl bis-(2-hydroxyethyl) sulfopropyl betaine; or an amphoteric surfactant, such as sodium 3-dodecylimino propionate or sodium 3-dodecyliminopropane sulphonate; or a non-ionic surfactant, preferably selected from the group consisting of C4-C22 alkyl ethoxylates with about 1-25 ethylene oxide units; ethoxylated/propoxylated akyl alcohols, such as [C10] deceth-n, laureth-n and trideceth-n, where n is an integer indicating the average number of ethylene oxide moieties in ethoxyl chain; castor oil ethoxylate, alkyl dialkyl amine oxides; alkanoyl glucose amides; ethoxylated sorbitol alkyl esters, such as sorbitol polyethylene glycol ethers, with 3 to 30 ethoxyl groups, esterified with oleic, myristic, stearic, palmitic acid, and the like; sorbitan alkylate, such as sorbitan palmitate, sorbitan oleate, and the like; polyoxyethylene sorbitan fatty acid esters, such as Polysorbate 20, Polysorbate 60 and Polysorbate 80; C10 to C18 alkyl amine oxides and C8 to C12 alkoxy ethyl dihydroxy ethyl amine oxides, such as N,N-dihydroxyethyl-N-stearamine oxide, ethoxylated lauramide and lauryldimethyl-amine oxide; cocamide diethanolamine, alkyl polyglycosides, such as glyceryl stearate, sucrose laurate and sucrose palmitate; ethoxylated alkyl glucose dialkyl esters, such as polyethyleneglycol-120 methyl glucose dioleate; mono- and dialkyl polyglycerides, such as octanoic acid hexaglyceryl ester, riccinoleic acid hexaglyceryl ester, cocoic acids, tetraglyceryl esters, caprylic/capric acid triglycerides; ethoxylated hydrogenated castor oil and sorbitan olivate, and cetearyl olivate;

Other suitable functional ingredients are vitamins. Illustrative vitamins include, but are not limited to vitamin C, vitamin F, vitamin K, folic acid. Derivatives of the vitamins may also be employed. In embodiments, the content of functional ingredient in the solid anhydrous composition is in the range of 0.15 wt% to 3 wt%, preferably in the range of about 0.15 wt% to 2 wt%, referred to the weight of the solid anhydrous composition.

ANTIOXIDANTS

As used herein, the term "antioxidants" is a chemical compound that slows down or completely prevents the oxidation of other substances. In embodiments, antioxidants include vitamin E (Tocopherol) and its derivatives, BHT or BHA. For instance, derivatives of vitamin E include tocopheryl acetate, tocopheryl palmitate and tocopheryl linoleate.

COLORANTS

As used herein, the term "colorant" refers to a substance that is added or applied in order to change the color of a material or a composition. Suitable colorants are pigments and dyes. Suitable pigments are organic and inorganic pigments, and can be metallic pigments or metal salts, mineral pigments like mica or synthetic mica, synthetic pigments or plant-derived or animal-derived pigments that are skin compatible.

The invention is not limited to the embodiments shown and described but also be defined by any other combination of specific features of all the individual features disclosed as a whole, provided that the individual features are not mutually exclusive, or a specific combination of individual features is not explicitly excluded.

## EXAMPLE 1

The sweating test of example 1 compares the anti-sweating effect of the dry mixture of the present invention with the anti-sweating effect of two mineral anti-sweating agents (hydrophilic fumed silica and modified hectorite organoclay). A lipstick without anti-sweating agent at all has also been prepared and serves as reference for the evaluation of sweating.

For the sweating test of example 1, four different lipsticks have been prepared with the same base ingredients and a different anti-sweating agent.

- The lipstick Nr. 1 comprises no anti-sweating agent.
- The lipstick Nr. 2 comprises 2% of dry mixture according to the claimed invention, commercialized as Celova® Care Powder (Weidmann) used as anti-sweating agent, wherein Celova Care Powder from Weidmann comprises 40-60% fibrous material.
- The lipstick Nr. 3 comprises 2% of modified hectorite organoclay commercialized as Bentone® 38V used as anti-sweating agent.
- The lipstick Nr. 4 comprises 2% of hydrophilic fumed silica commercialized as Aerosil® 200, used as anti-sweating agent.

**Table 1**

| | | **Sweating measurements of lipsticks Nr. 1, Nr. 2, Nr. 3 and Nr. 4** | | | |
|---|---|---|---|---|---|
| **Figure** | **Time in the oven at 45°C** | **Lipstick Nr. 1 (no sweating agent)** | **Lipstick Nr. 2 (1% Celova® Care Powder)** | **Lipstick Nr. 3 (1% Bentone® 38V)** | **Lipstick Nr. 4 (1% Aerosil® 200)** |
| **Fig. 2** | **0'** | All the sticks have been poured in a mold at 80°C, cooled down at 4°C for 10 min and let at room temperature for 4 days before the start of the test.<br>At 25°C their surfaces are mat and uniform, there are dry. | | | |
| | **15'** | Small oil drops only on the stick without powder | The surface is as dry as at room temperature. | The surface is as dry as at room temperature. | The surface is as dry as at room temperature. |
| | **30'** | Greasy appearance, there is a layer of oil on the surface | Dry and mat surface | Greasy appearance, there is a layer of oil on the surface | Greasy appearance, there is a layer of oil on the surface |
| | **1 h** | Shining area on the top surface, representing a large drop of liquid. The right side of the lipstick looks also shiny.<br>A part of the lipid has melted und has separated from the lipstick composition. | Dry and mat surface. | Shining body | Shining body |

(continued)

| | | Sweating measurements of lipsticks Nr. 1, Nr. 2, Nr. 3 and Nr. 4 | | | |
|---|---|---|---|---|---|
| **Figure** | **Time in the oven at 45°C** | **Lipstick Nr. 1 (no sweating agent)** | **Lipstick Nr. 2 (1% Celova® Care Powder)** | **Lipstick Nr. 3 (1% Bentone® 38V)** | **Lipstick Nr. 4 (1% Aerosil® 200)** |
| | **2 h** | Completely sweaty surfaces | Dry and mat surface | Shining and greasy surfaces | Shining and greasy surfaces |
| | **4 h** | Completely sweaty surfaces | Dry and mat surface | Shining and greasy surfaces | Shining and greasy surfaces |
| **Fig. 3** | **8 h** | Completely sweaty surfaces | Dry and mat surface | Shining and greasy surfaces | Shining and greasy surfaces |

CONCLUSION OF THE SWEATING TEST OF EXAMPLE 1:

- The lipstick Nr. 1 that does not comprise any anti-sweating agent starts losing a part of the composition that has migrated on its surface already after 15 minutes. After 60 minutes, a shining area formed on the top surface. It is completely sweaty after two hours.
- Lipstick Nr. 2 comprising the dry mixture according to the present invention has the same mat and dry surface after 2 hours at 45°C, as before the heat exposure.
- The lipstick Nr. 3 and lipstick Nr. 4 with mineral based anti-sweating agent (Bentone® 38V and Aerosil® 200) seem to have lost less of their composition than lipstick Nr. 1, but the shiny surface shows that a part of the composition has separated anyway.

Consequently, the dry mixture of the present invention has an improved anti-sweating effect relative to modified hectorite organoclay (Bentone® 38V) and hydrophilic fumed silica (Aerosil® 200) in the tested lipstick compositions.

**EXAMPLE 2**

The sweating test of example 2 compares the anti-sweating effect of the dry mixture of the present invention with the anti-sweating effect of chemically modified cellulose (Ethocel™). A lipstick without anti-sweating agent at all has also been prepared and serves as reference for the evaluation of the sweating.

For the sweating test of example 2, three different lipsticks have been prepared with the same ingredients listed as ingredients A, B and C in table 2, and a different anti-sweating agent (ingredient D):

- The lipstick Nr. 5 does not comprise any anti-sweating agent.
- The lipstick Nr. 6 comprises 2% of Celova® Care (Weidmann), a non-chemically modified anti-sweating agent according to the present invention.
- The lipstick Nr. 7 comprises 2% Ethocel™ Standard 100 Premium (Cellulosic Industrial), a chemically modified anti-sweating agent.

**Table 2**

| Compositions of lipstick Nr. 5, lipstick Nr. 6, and Lipstick Nr. 7 (%wt) | | | | | |
|---|---|---|---|---|---|
| **IINGREDIENT GROUP** | **INGREDIENT** | **TRADE NAME** | **LIPSTICK NR. 5** | **LIPSTICK NR. 6** | **LIPSTICK NR. 7** |
| A | Oryza Sativa (Rice) Bran Wax | Rice Wax – Kahlwax 2811 | 4,0 | 4,0 | 4,0 |
| | Helianthus Annuus Seed Cera, Ascorbyl Palmitate, Tocopherol | Sunflower Wax – Kahlwax 6607L | 6,0 | 6,0 | 6,0 |
| | Copernicia Cerifera Cera | Carnauba T1 Wax | 9,0 | 9,0 | 9,0 |
| | Euphorbia Cerifera Wax | Candelilla Wax - Kahlwax 2039L | 5,0 | 5,0 | 5,0 |
| B | Pentaerythrityl Tetraisostearate | Zetemol 5418 | 15,0 | 15,0 | 15,0 |
| | Dicaprylyl Carbonate | Cetiol CC | 17,3 | 15,3 | 15,3 |
| | Coco-Caprylate | Cetiol C5 | 16,0 | 16,0 | 16,0 |
| | Bis-Diglyceryl Polyacyladipate-2 | Supersoft Ester – Kahlwax 6421 | 2,0 | 2,0 | 2,0 |
| | Ricinus Communis Seed Oil | Castor oil | 5,5 | 5,5 | 5,5 |
| | Tocopherol | Tocopherol | 0,2 | 0,2 | 0,2 |
| C | Caprylic/Capric Triglyceride, CI45410, CI42090, CI15850 | Pigment dispersion – dark Red | 20,0 | 20,0 | 20,0 |
| D | Anti-sweating agent: dry mixture according to the present invention | Celova® Care Powder | - | **2%** | - |
| | Anti-sweating agent: Chemically modified cellulose | ETHOCEL™ STANDARD PREMIUM 100 | - | - | **2%** |
| | **Total** | | **100%** | **100%** | **100%** |

PREPARATION

Lipstick Nr. 5, lipstick Nr. 6 and lipstick Nr. 7 have been produced each with the following steps, wherein the ingredients listed in table 2 have been processed in four ingredient groups, ingredients A, ingredients B, ingredients C and ingredients D as described in table 2:

- Step 1: The ingredients of group A (see table 2) have been heated to 85°C under stirring.
- Step 2: The ingredients of group B have been heated to 80°C, added to the ingredients of group A and then mixed.
- Step 3: The pigment dispersion comprising Caprylic/Capric Triglyceride of group C has been mixed and added to the mixture of ingredients A and B, and then mixed with a homogenizer IKA-ULTRATURRAX T 25 DIGITAL - S25N - 25 F.
- Step 4: The whole formula (ingredients A, B and C) has been passed through three roll mills.
- Step 5: The anti-sweating agent of ingredient group D has been added and mixed with the homogenizer IKA-ULTRATURRAX T 25 DIGITAL - S25N - 25 F until complete dispersion.
- Step 6: The mass has been poured in a lipstick mold at 80°C.
- Step 7: The mold has been put for 10 minutes at 4°C.

- Step 8: The lipstick has been taken out of the mold.
- Step 9: 4 days have been waited before carrying out the sweating test.

SWEATING TEST OF EXAMPLE 2

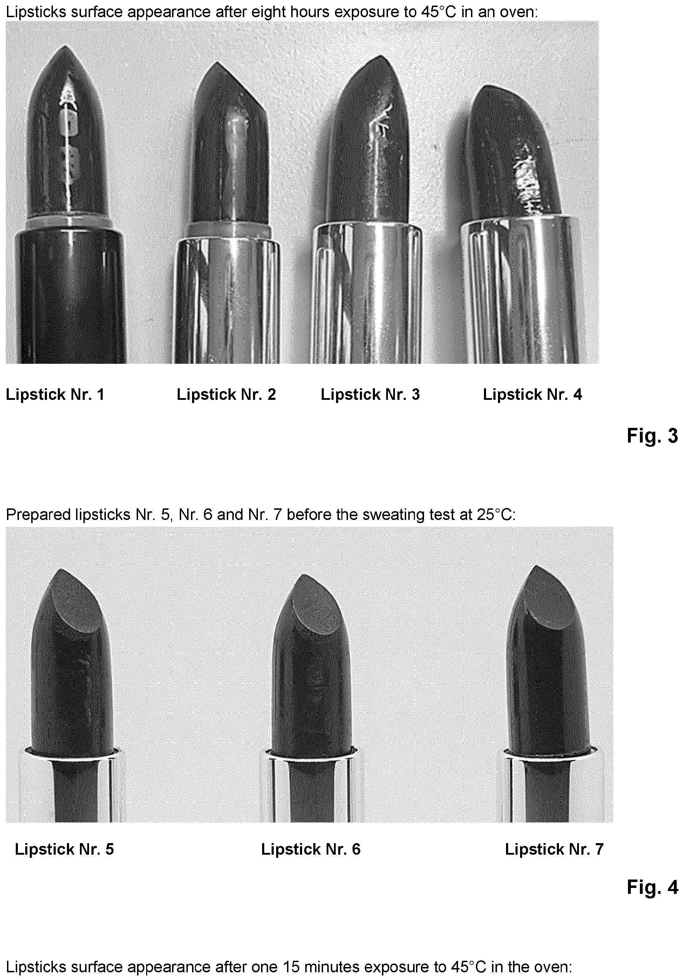

As shown on Fig. 4, the lipsticks have a dry surface at room temperature before starting the sweating test.
The sweating tests have been carried out by operating of the following steps:

- Lipsticks Nr. 5, 6 and 7 have been put in an oven MEMMERT 30 -1060 at 45°C for 6 hours.
- The surfaces of the lipsticks have been observed and photographed after they have stayed in the oven at 45°C for 15 minutes, 30 minutes, 1 hour, 2 hours, 4 hours, and 6 hours.

OBSERVATIONS:

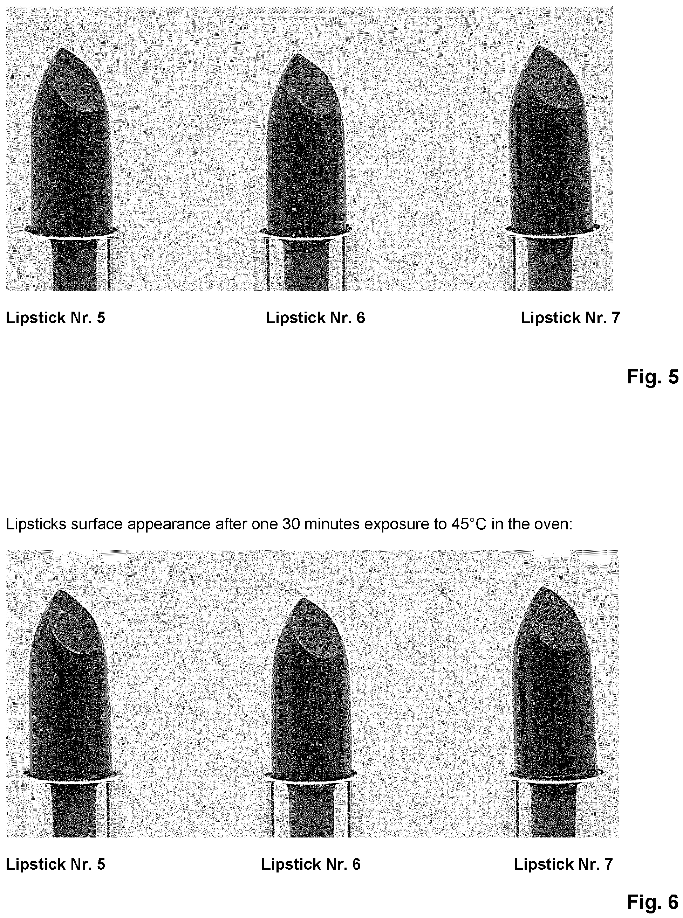

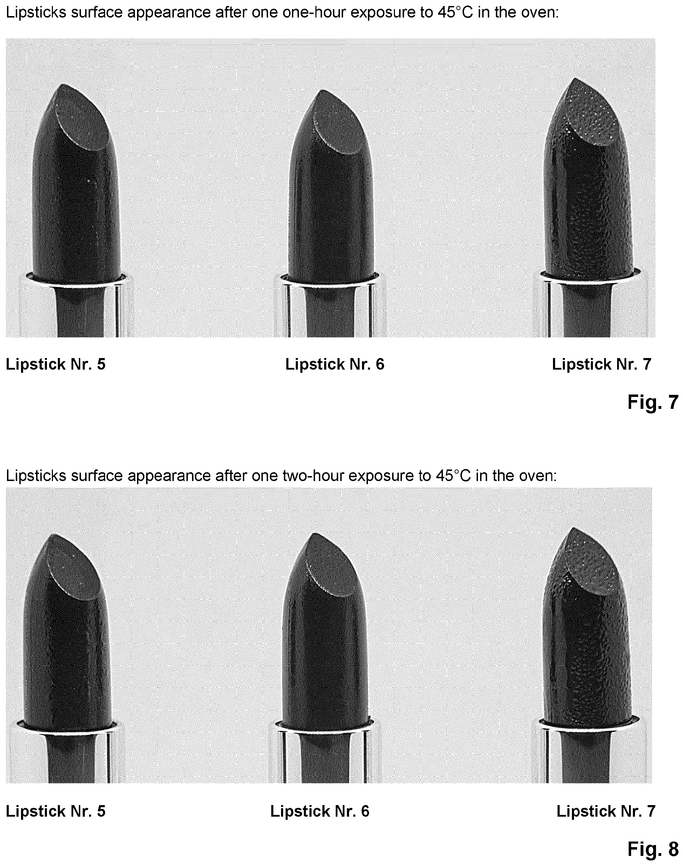

The results of the sweating test of example 2 are shown in Fig. 5, Fig. 6, Fig. 7 and Fig. 8 and in table 3.
For each time spent at 45°C, the visual appearance of the surface of the lipsticks in regard to the amount and form of the liquids that have exuded on the lipstick surfaces is described in table 3. The figures Fig. 5, Fig. 6, Fig. 7 and Fig. 8 show the appearances of lipsticks Nr. 5, Nr. 6 and Nr. 7 after 15 minutes, 30 minutes, 1 hour and 2 hours heat exposure (at 45°C).

**Table 3**

| **Sweating measurements of lipsticks Nr. 5, Nr. 6 and Nr. 7** | | | | |
|---|---|---|---|---|
| **Figure** | **Time in the oven at 45°C** | **Lipstick Nr. 5** | **Lipstick Nr. 6 (2% Celova® Care Powder)** | **Lipstick Nr. 7 (2% Ethocel™ standard premium 100)** |
| | **0'** | All the sticks have been poured in a mold at 80°C, cooled down at 4°C for 10 min and let at room temperature for 4 days before the start of the test.<br>At 25°C the surface is dry, there are no drops of oil | | |
| **Fig. 5** | **15'** | Small oil droplets have appeared on the surface of the top. | Dry and mat surface | Small oil droplets have appeared on the surface on the top and on the body of the stick. |
| **Fig. 6** | **30'** | Oil pouring on the top and small droplets on the body. | Dry and mat surface | Droplets of bigger size on the top and on the body. |
| **Fig. 7** | **1 h** | Oil pouring on the top and bigger droplets on the body. | Dry and mat surface | Very big droplets on the top and on the body. |
| **Fig. 8** | **2 h** | Oil pouring on the top and bigger droplets on the body. | Dry and mat surface | Very big droplets on the top and on the body, |
| | **4 h** | Oil pouring on the top and bigger droplets on the body. | Dry and mat surface | Very big droplets on the top and on the body, |
| | **6 h** | Oil pouring on the top and bigger droplets on the body. | Dry and mat surface | Very big droplets on the top and on the body, |

CONCLUSION OF EXAMPLE 2:

Lipstick Nr. 5, which does not comprise any anti-sweating agent, presents large drops of liquid on its top surface and on the side surfaces.
Lipstick Nr. 6 with the dry mixture of the present invention has an unchanged surface over the six hours spent at 45°C. It

stays mat and dry.

Lipstick Nr. 7 with chemically modified cellulose (Ethocel™ Standard Premium 100) releases an increasing number of small droplets over its entire surface.

Consequently, the use of the dry mixture according to the present invention is more efficient as an anti-sweating agent in a lipstick with the compositions described in table 2 than chemically modified cellulose.

**EXAMPLE 3**

The sweating test of example 3 compares the anti-sweating effect of 0%, 1% and 2% of the dry mixture of the present invention .

For the sweating test of example 3, three different lipsticks have been prepared with the same ingredients listed as ingredients A, B and C in table 4, and a different amount of Celova® Care (Weidmann) as anti-sweating agent (ingredient D):

- Lipstick Nr. 8 does not comprise any anti-sweating agent: 0% Celova® Care (Weidmann).
- Lipstick Nr. 9 comprises 1% Celova® Care (Weidmann), a non-chemically modified anti-sweating agent according to the present invention.
- Lipstick Nr. 10 comprises 2% Celova® Care (Weidmann), the same non-chemically modified anti-sweating agent according to the present invention.

**Table 4**

**Compositions of lipstick Nr. 8, lipstick Nr. 9, and lipstick Nr. 10 (%wt)**

| INGREDIENT GROUP | INGREDIENT | TRADE NAME | LIPSTICK NR. 8 | LIPSTICK NR. 9 | LIPSTICK NR. 10 |
|---|---|---|---|---|---|
| A | Oryza Sativa (Rice) Bran Wax | Rice Wax – Kahlwax 2811 | 4,0 | 4,0 | 4,0 |
| | Helianthus Annuus Seed Cera, Ascorbyl Palmitate, Tocopherol | Sunflower Wax – Kahlwax 6607L | 6,0 | 6,0 | 6,0 |
| | Copernicia Cerifera Cera | Carnauba T1 Wax | 9,0 | 9,0 | 9,0 |
| | Euphorbia Cerifera Wax | Candelilla Wax - Kahlwax 2039L | 5,0 | 5,0 | 5,0 |
| B | Pentaerythrityl Tetraisostearate | Zetemol 5418 | 15,0 | 15,0 | 15,0 |
| | Dicaprylyl Carbonate | Cetiol CC | 17,3 | 16,3 | 15,3 |
| | Coco-Caprylate | Cetiol C5 | 16,0 | 16,0 | 16,0 |
| | Bis-Diglyceryl Polyacyladipate-2 | Supersoft Ester – Kahlwax 6421 | 2,0 | 2,0 | 2,0 |
| | Ricinus Communis Seed Oil | Castor oil | 5,5 | 5,5 | 5,5 |
| | Tocopherol | Tocopherol | 0,2 | 0,2 | 0,2 |
| C | Caprylic/Capric Triglyceride, CI45410, CI42090, CI15850 | Pigment dispersion – dark Red | 20,0 | 20,0 | 20,0 |
| D | Anti-sweating agent: dry mixture according to the present invention | Celova Care Powder | - | **1%** | **2%** |
| | **Total** | | **100%** | **100%** | **100%** |

PREPARATION

Lipstick Nr. 8, lipstick Nr. 9 and lipstick Nr. 10 have been produced each with the following steps, wherein the ingredients listed in table 4 have been processed in four ingredient groups, ingredients A, ingredients B, ingredients C and ingredients D as described in table 4:

- Step 1: The ingredients of group A (see table 4) have been heated to 85°C under stirring.
- Step 2: The ingredients of group B have been heated to 80°C, added to the ingredients of group A and then mixed.
- Step 3: The pigment dispersion comprising Caprylic/Capric Triglyceride of group C has been mixed and added to the mixture of ingredients A and B, and then mixed with a homogenizer IKA-ULTRATURRAX T 25 DIGITAL - S25N - 25 F.
- Step 4: The whole formula (ingredients A, B and C) has been passed through three roll mills.
- Step 5: The anti-sweating agent of ingredient group D has been added and mixed with the homogenizer IKA-ULTRATURRAX T 25 DIGITAL - S25N - 25 F until complete dispersion.
- Step 6: The mass has been poured in a lipstick mold at 80°C.
- Step 7: The mold has been cooled down for 10 minutes at 4°C.
- Step 8: The lipstick has been taken out from the mold.
- Step 9: 4 days have been waited before carrying out the sweating test.

SWEATING TEST OF EXAMPLE 3

Before the sweating test, the lipsticks have a dry surface at room temperature.

The sweating tests have been carried out by carrying out the following steps:
Lipsticks Nr. 8, 9 and 10 have been put in an oven MEMMERT 30 -1060 at 45°C for 8 hours. The surfaces of the lipsticks have been observed and photographed after they have stayed in the oven at 45°C for 15 minutes, 30 minutes, 1 hour, 2 hours, 4 hours and 8 hours.

OBSERVATION

The results of the sweating test of example 3 are shown in table 5.

For each time spent in the 45°C warm oven, the visual appearance of the surface of the lipsticks in regard to the amount and form of the liquids that have exuded on the lipstick surfaces is described in table 5.

**Table 5**

| **Sweating measurements of lipsticks Nr. 8, Nr. 9 and Nr. 10** | | | |
|---|---|---|---|
| **Time in the oven at 45°C** | **Lipstick Nr. 8 (without anti-sweating agent)** | **Lipstick Nr. 9 (1% Celova® Care Powder)** | **Lipstick Nr. 10 (2% Celova® Care Powder)** |
| **0'** | All the sticks have been poured in a mold at 80°C, cooled down at 4°C for 10 min and let at room temperature for 4 days before the start of the test. At 25°C the surface is dry, there are not drops of oil | | |
| **15'** | Small oil droplets have appeared on the surface of the top. | Dry and mat surface | Dry and mat surface |
| **30'** | Oil pouring on the top and small droplets on the body. | Dry and mat surface | Dry and mat surface |
| **1h** | Oil pouring on the top and small droplets on the body. | Dry and mat surface | Dry and mat surface |
| **2h** | Oil pouring on the top and bigger droplets on the body. | Dry and mat surface | Dry and mat surface |
| **4h** | Oil pouring on the top and bigger droplets on the body. | Dry and mat surface | Dry and mat surface |
| **8h** | Oil pouring on the top and bigger droplets on the body. | Dry and mat surface | Dry and mat surface |

CONCLUSION OF EXAMPLE 3

Both 1% and 2% of the dry mixture according to the present invention in the lipstick compositions described in table 5 have an anti-sweating effect.

## Claims

1. Use of a dry mixture comprising at least one polyol and a fibrous material as an anti-sweating-agent in a solid anhydrous composition, wherein the fibrous material is a chemically unmodified plant pulp comprising cellulose, wherein the fibrous material contains more than 10 wt% xylose referred to the weight of the fibrous material.

2. The use according to one of the preceding claims, **characterized in that** the solid anhydrous composition is a personal care composition selected from skin care, color cosmetic, and hair care, or a material care composition selected from leather greases, ski waxes, wood furniture protection and anti-seizing compositions.

3. The use according to one of the preceding claims, **characterized in that** the at least one polyol is selected from the group consisting of glycerol, erythritol, threitol, pentaerythritol, xylitol, ribitol, arabinitol, mannitol, sorbitol, allitol, altritol, galactitol, glucitol, iditol, xylitol and a mixture thereof.

4. The use according to one of the preceding claims, **characterized in that** the weight ratio of the polyol to the fibrous material in the dry mixture is in the range of 0.5 to 1.5.

5. The use according to one of the preceding claims, **characterized in that** the plant pulp is from Eucalyptus tree, beech tree, birch tree, aspen tree, poplar tree or oak tree.

6. The use according to one of the preceding claims, **characterized in that** the fibrous material comprises micro-scaled fibril agglomerates with an average length in the range of 500 nm to 1000 μm.

7. The use according to one of the preceding claims, **characterized in that** the water activity of the dry mixture is lower than 0.6 at room temperature.

8. The use according to one of the preceding claims, **characterized in that** the dry mixture is in powder form or in flake form.

9. A solid anhydrous composition comprising:

    - lipids that are solid at room temperature in the range of 20 wt% to 50 wt% of the weight of the solid anhydrous composition,
    - lipids that are fluid at room temperature in the range of 30 wt% to 80 wt% of the weight of the solid anhydrous composition,
    - at least 0.1 wt% of a dry mixture comprising at least one polyol and a fibrous material,

        wherein the fibrous material is a chemically unmodified plant pulp comprising cellulose,
        wherein the fibrous material contains more than 10 wt% xylose referred to the weight of the fibrous material.

10. The solid anhydrous composition according to claim 9 further comprising at least one colorant in the range of 5 wt% to 15 wt% of the weight of the solid anhydrous composition.

11. The solid anhydrous composition according to claim 9 or 10 further comprising at least one functional ingredient.

12. The solid anhydrous composition according to any one of claims 9 to 11, **characterized in that** the fibrous material comprises micro-scaled fibril agglomerates with an average length in the range of 500 nm to 1000 μm.

13. The solid anhydrous composition according to any one of claims 9 to 12, **characterized in that** the solid anhydrous composition is a personal care composition selected from skincare, hair care and color cosmetic, or a material care composition selected from leather greases, ski waxes, wood furniture protection and anti-seizing compositions.

**14.** A method for preparing a solid anhydrous composition according to any one of claims 9 to 13 comprising at least the following steps:

a) Heating up lipids that are solid at room temperature to a temperature in the range of 75°C to 90°C,
b) Mixing lipids that are fluid at room temperature to the heated lipids of step a) , and cooling down to a temperature in the range of 70°C to 80°C,
c) Adding a dry mixture comprising at least one polyol and a fibrous material to the lipids,

wherein the fibrous material is a chemically unmodified plant pulp comprising cellulose,
wherein the fibrous material contains more than 10 wt% xylose referred to the weight of the fibrous material,

d) Mixing with a homogenizer until a homogeneous composition is obtained, and
e) Cooling down the homogeneous composition .

**15.** The method according to claim 14, **characterized in that** after step a), b) or c) at least one colorant and/or at least one functional ingredient are added to the lipids and mixed with a homogenizer.

**Amended claims in accordance with Rule 137(2) EPC.**

**1.** Use of a dry mixture comprising at least one polyol and a fibrous material as an anti-sweating-agent in a solid anhydrous composition, wherein the fibrous material is a chemically unmodified plant pulp comprising cellulose, wherein the fibrous material contains more than 10 wt% xylose referred to the weight of the fibrous material.

**2.** The use according to one of the preceding claims, **characterized in that** the solid anhydrous composition is a personal care composition selected from skin care, color cosmetic, and hair care.

**3.** The use according to one of the preceding claims, **characterized in that** the at least one polyol is selected from the group consisting of glycerol, erythritol, threitol, pentaerythritol, xylitol, ribitol, arabinitol, mannitol, sorbitol, allitol, altritol, galactitol, glucitol, iditol, xylitol and a mixture thereof.

**4.** The use according to one of the preceding claims, **characterized in that** the weight ratio of the polyol to the fibrous material in the dry mixture is in the range of 0.5 to 1.5.

**5.** The use according to one of the preceding claims, **characterized in that** the plant pulp is from Eucalyptus tree, beech tree, birch tree, aspen tree, poplar tree or oak tree.

**6.** The use according to one of the preceding claims, **characterized in that** the fibrous material comprises micro-scaled fibril agglomerates with an average length in the range of 500 nm to 1000 μm.

**7.** The use according to one of the preceding claims, **characterized in that** the dry mixture has a solid content in the range of 70% to 100%, more preferably in the range of 80 wt% to 99 wt%, even more preferably in the range of 90 wt% to 97 wt%, referred to the total weight of the dry mixture.

**8.** The use according to one of the preceding claims, **characterized in that** the dry mixture is in powder form or in flake form.

**9.** A solid anhydrous composition comprising:

- lipids that are solid at room temperature in the range of 20 wt% to 50 wt% of the weight of the solid anhydrous composition,
- lipids that are fluid at room temperature in the range of 30 wt% to 80 wt% of the weight of the solid anhydrous composition,
- at least 0.1 wt% of a dry mixture comprising at least one polyol and a fibrous material ,

wherein the fibrous material is a chemically unmodified plant pulp comprising cellulose,
wherein the fibrous material contains more than 10 wt% xylose referred to the weight of the fibrous material.

**10.** The solid anhydrous composition according to claim 9 further comprising at least one colorant in the range of 5 wt% to

15 wt% of the weight of the solid anhydrous composition.

**11.** The solid anhydrous composition according to claim 9 or 10 further comprising at least one functional ingredient.

**12.** The solid anhydrous composition according to any one of claims 9 to 11, **characterized in that** the fibrous material comprises micro-scaled fibril agglomerates with an average length in the range of 500 nm to 1000 μm.

**13.** The solid anhydrous composition according to any one of claims 9 to 12, **characterized in that** the solid anhydrous composition is a personal care composition selected from skincare, hair care and color cosmetic, or a material care composition selected from leather greases, ski waxes, wood furniture protection and anti-seizing compositions.

**14.** A method for preparing a solid anhydrous composition according to any one of claims 9 to 13 comprising at least the following steps:

a) Heating up lipids that are solid at room temperature to a temperature in the range of 75°C to 90°C,
b) Mixing lipids that are fluid at room temperature to the heated lipids of step a) , and cooling down to a temperature in the range of 70°C to 80°C,
c) Adding a dry mixture comprising at least one polyol and a fibrous material to the lipids,

wherein the fibrous material is a chemically unmodified plant pulp comprising cellulose,
wherein the fibrous material contains more than 10 wt% xylose referred to the weight of the fibrous material,

d) Mixing with a homogenizer until a homogeneous composition is obtained, and
e) Cooling down the homogeneous composition .

**15.** The method according to claim 14, **characterized in that** after step a), b) or c) at least one colorant and/or at least one functional ingredient are added to the lipids and mixed with a homogenizer.

10 μm

Fig. 1

Prepared lipsticks Nr. 1, Nr. 2, Nr. 3 and Nr. 4 before the sweating test at 25°C:

Lipstick Nr. 1 Lipstick Nr. 2 Lipstick Nr. 3 Lipstick Nr. 4

Fig. 2

Lipsticks surface appearance after eight hours exposure to 45°C in an oven:

**Lipstick Nr. 1** **Lipstick Nr. 2** **Lipstick Nr. 3** **Lipstick Nr. 4**

**Fig. 3**

Prepared lipsticks Nr. 5, Nr. 6 and Nr. 7 before the sweating test at 25°C:

**Lipstick Nr. 5** **Lipstick Nr. 6** **Lipstick Nr. 7**

**Fig. 4**

Lipsticks surface appearance after one 15 minutes exposure to 45°C in the oven:

Lipstick Nr. 5 Lipstick Nr. 6 Lipstick Nr. 7

Fig. 5

Lipsticks surface appearance after one 30 minutes exposure to 45°C in the oven:

Lipstick Nr. 5 Lipstick Nr. 6 Lipstick Nr. 7

Fig. 6

Lipsticks surface appearance after one one-hour exposure to 45°C in the oven:

**Lipstick Nr. 5** **Lipstick Nr. 6** **Lipstick Nr. 7**

**Fig. 7**

Lipsticks surface appearance after one two-hour exposure to 45°C in the oven:

**Lipstick Nr. 5** **Lipstick Nr. 6** **Lipstick Nr. 7**

**Fig. 8**

Europäisches
Patentamt
European
Patent Office
Office européen
des brevets

# EUROPEAN SEARCH REPORT

Application Number
EP 25 16 5326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2023/143339 A1 (MANSER JULIA [CH] ET AL) 11 May 2023 (2023-05-11)<br>* paragraph [0001] *<br>* paragraph [0046] *<br>* paragraph [0043] *<br>* example 1 *<br>* examples 2-5 *<br>----- | 1-15 | INV.<br>A61K8/02<br>A61K8/34<br>A61K8/60<br>A61K8/73<br>A61K8/9789<br>A61Q1/06<br>C09D191/00<br>C09G3/00<br>C14C9/02 |
| Y | US 2015/110841 A1 (WIECHERS SUSANN [DE] ET AL) 23 April 2015 (2015-04-23)<br>* paragraph [0001] *<br>* claim 1 *<br>* Eyeshadow formulation; paragraph [0089] *<br>----- | 1-15 | |
| A | US 2015/297469 A1 (HAYASHI HISATO [JP] ET AL) 22 October 2015 (2015-10-22)<br>* paragraph [0001] *<br>* paragraph [0015] *<br>* Production example 2 *<br>* example 8; table 3 *<br>----- | 1-15 | TECHNICAL FIELDS SEARCHED (IPC) |
| A | US 2021/010201 A1 (TRUNIGER STEFAN [CH] ET AL) 14 January 2021 (2021-01-14)<br>* paragraph [0013] *<br>* paragraph [0062] *<br>* paragraph [0110] *<br>* paragraph [0117] *<br>----- | 1-15 | A61K<br>A61Q<br>C09G<br>C08L<br>C09D<br>C14C |
| A | WO 2023/187180 A1 (MINASOLVE SAS [FR]) 5 October 2023 (2023-10-05)<br>* page 1 *<br>* example 4; table 4 *<br>----- | 1-15 | |

-/--

The present search report has been drawn up for all claims

2

| Place of search | Date of completion of the search | Examiner |
|---|---|---|
| Munich | 19 September 2025 | Durand-Oral, Ilknur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 25 16 5326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2024/014166 A1 (SANO CO LTD [JP]; DAIO SEISHI KK [JP]) 18 January 2024 (2024-01-18) * paragraph [0008] * * paragraph [0032] * * paragraph [0115] * * Powder composition 1; table 2 * ----- | 1-15 | |

| | TECHNICAL FIELDS SEARCHED (IPC) |
|---|---|
| | |

The present search report has been drawn up for all claims

2

| Place of search | Date of completion of the search | Examiner |
|---|---|---|
| Munich | 19 September 2025 | Durand-Oral, Ilknur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 16 5326

This annex lists the patent family members relating to the patent documents cited in the above-mentioned European search report. The members are as contained in the European Patent Office EDP file on
The European Patent Office is in no way liable for these particulars which are merely given for the purpose of information.

19-09-2025

| Patent document cited in search report | Publication date | Patent family member(s) | Publication date |
|---|---|---|---|
| US 2023143339 A1 | 11-05-2023 | BR 112022018383 A2 | 08-11-2022 |
| | | CN 115484916 A | 16-12-2022 |
| | | EP 4138765 A1 | 01-03-2023 |
| | | US 2023143339 A1 | 11-05-2023 |
| | | WO 2021214300 A1 | 28-10-2021 |
| US 2015110841 A1 | 23-04-2015 | CN 104144673 A | 12-11-2014 |
| | | DE 102012203307 A1 | 05-09-2013 |
| | | EP 2819640 A2 | 07-01-2015 |
| | | US 2015110841 A1 | 23-04-2015 |
| | | WO 2013127598 A2 | 06-09-2013 |
| US 2015297469 A1 | 22-10-2015 | CN 104955440 A | 30-09-2015 |
| | | EP 2929874 A1 | 14-10-2015 |
| | | JP 2018109030 A | 12-07-2018 |
| | | JP WO2014088034 A1 | 05-01-2017 |
| | | US 2015297469 A1 | 22-10-2015 |
| | | WO 2014088034 A1 | 12-06-2014 |
| US 2021010201 A1 | 14-01-2021 | EP 3607136 A1 | 12-02-2020 |
| | | US 2021010201 A1 | 14-01-2021 |
| | | WO 2018185227 A1 | 11-10-2018 |
| WO 2023187180 A1 | 05-10-2023 | EP 4504128 A1 | 12-02-2025 |
| | | US 2025213437 A1 | 03-07-2025 |
| | | WO 2023187180 A1 | 05-10-2023 |
| WO 2024014166 A1 | 18-01-2024 | CN 119384267 A | 28-01-2025 |
| | | EP 4527468 A1 | 26-03-2025 |
| | | JP 7555079 B2 | 24-09-2024 |
| | | JP 2024010723 A | 25-01-2024 |
| | | JP 2024107247 A | 08-08-2024 |
| | | KR 20250037417 A | 17-03-2025 |
| | | WO 2024014166 A1 | 18-01-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1134170 A
- WO 9511000 A1
- EP 3606500 B1


### Non-patent literature cited in the description


- **HOSSAIN ME** ; **KHAN MI** ; **KETATA C** ; **ISLAM R**. Comparative pathway analysis of paraffin wax and bee wax for industrial application. *Journal of Characterization and Development of Novel Materials*, 2012, vol. 1 (4), 1-13
- **BADING M** ; **OLSSON O** ; **KÜMMERER K**. Analysis of environmental biodegradability of cellulose-based pharmaceutical excipients in aqueous media. *Chemosphere*, 2024, vol. 352, 141298
- OECD 301 Ready Biodegradability. *OECD Guidelines for the Testing of Chemicals*, 1992
- Dreidimensionale Strukturanalyse und Modellierung des Kraft-Dehnungsverhaltens von Fasergefügen. **WOLFINGER T**. Dissertation. TU Dresden, Fakultät Umweltwissenschaften, 2016